# FASCICULE DE BREVET EUROPEEN

(11) **EP 1 098 901 B1**
(45) Date de publication et mention de la délivrance du brevet: **15.01.2003**
(21) Numéro de dépôt: 99932916.2
(22) Date de dépôt: 20.07.1999
(51) Int. Cl.: C07H 17/08, A61K 31/70

(54) **NOUVEAUX DERIVES DE LA 6-DEOXY ERYTHROMYCINE, LEUR PROCEDE DE PREPARATION ET LEUR APPLICATION COMME MEDICAMENTS**
6-DEOXY-ERYTHROMYCIN-DERIVATE, IHRER HERSTELLUNG, UND IHRER VERWENDUNG ALS ARZNEIMITTELN
NOVEL 6-DEOXY ERYTHROMYCIN DERIVATIVES, METHOD FOR PREPARING SAME AND USE AS MEDICINES

(30) Priorité: 21.07.1998 FR 9809259
(43) Date de publication de la demande: 16.05.2001
(73) Titulaire: Aventis Pharma S.A., 92160 Antony (FR)
(72) Inventeur: AUGER, Jean-Michel, Décédé. (FR); DENIS, Alexis, F-75011 Paris (FR)
(74) Mandataire: Vieillefosse, Jean-Claude
(86) Numéro de dépôt international: FR9901769
(87) Numéro de publication internationale: WO00005239

(56) Documents cités:
- WO-A-97/31929
- WO-A-97/42205
- WO-A-98/03530
- FR-A- 2 692 579
- G.GRIESGRABER ET AL.: "3-Keto-11,12-Carbazate Derivatives of 6-O-Methylerythromycin A Synthesis and In Vitro Activity." JOURNAL OF ANTIBIOTICS., vol. 49, no. 5, mai 1996 (1996-05), pages 465-477, XP002104319 TOKYO JP

## Description

La présente invention concerne de nouveaux dérivés de la 6-déoxy érythromycine, leur procédé de préparation et leur application comme médicaments.

On connait des dérivés 10-méthyl 6-méthoxy 3-oxo de l'érythromycine (cf WO 96/03530). On connaît également des dérivés de la 6-déméthoxy 10-déméthyle érythromycine (cf FR A-2692579). On connaît également des dérivés de l'érythromycine 3, 11, 12-trihydroxy utiles comme intermédiaires dans la synthèse de produits antibiotiques (cf WO 97/42205).

L'invention a pour objet les composés de formule (I) 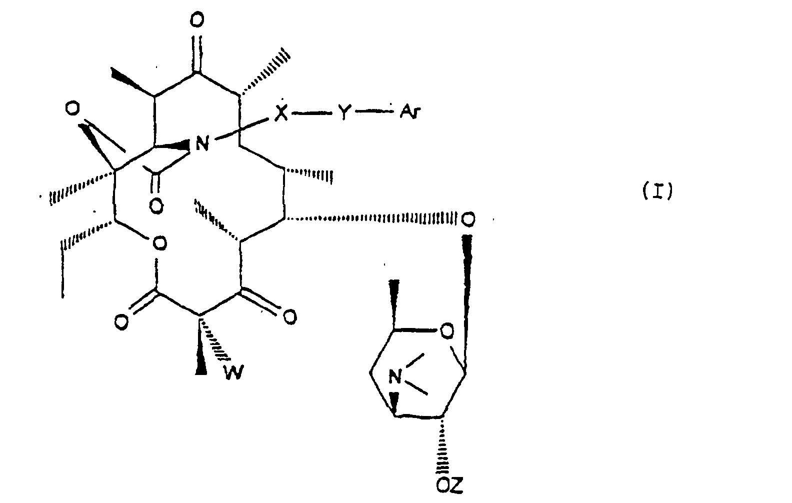 dans lesquels
- X représente un radical (NH)a, CH₂ ou SO₂ ou un atome d'oxygène, a représente le nombre 0 ou 1,
- Y représente un radical (CH₂)m-(CH=CH)n-(CH₂)o avec m+n+o ≤8, n = O ou 1,
- Ar représente un radical aryle ou hétéroaryle, éventuellement substitué
- W représente un atome d'hydrogène ou un atome d'halogène
- Z représente un atome d'hydrogène ou le reste d'un acide ainsi que leurs sels d'addition avec les acides.

Parmi les sels d'addition avec les acides, on peut citer les sels formés avec les acides acétique, propionique, trifluoroacétique, maléique, tartrique, méthanesulfonique, benzènesulfonique, p-toluènesulfonique et spécialement les acides stéarique, éthylsuccinique, ou laurylsulfonique.

Le radical aryle est de préférence un radical phényle ou naphtyle.

Le radical hétéroaryle substitué ou non peut-être le radical thiényle, furyle, pyrolyle, thiazolyle, oxazolyle, imidazolyle, par exemple le radical 4-(3-pyridinyl) 1H-imidazolyle, thiadiazolyle, pyrazolyle ou isopyrazolyle, un radical pyridyle, pyrimidyle, pyridazinyle ou pyrazinyle, ou encore un radical indolyle, benzofurannyle, benzothiazyle ou quinoléinyle.

Ces radicaux aryles peuvent comporter un ou plusieurs groupements choisis dans le groupe constitué par les radicaux hydroxyle, les atomes d'halogène, les radicaux NO₂, les radicaux C≡N, les radicaux alkyle, alkényle ou alkynyle, O-alkyle, O-alkényle ou O-alkynyle, S-alkyle, S-alkényle ou S-alkynyle et N-alkyle, N-alkényle ou N-alkynyle, renfermant jusqu'à 12 atomes de carbone éventuellement substitués par un ou plusieurs atomes d'halogène, le radical 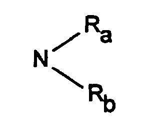 Rₐ et R_{b} identiques ou différents représentant un atome d'hydrogène ou un radical alkyle renfermant jusqu'à 12 atomes de carbone, le radical 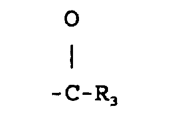 R₃ représentant un radical alkyle renfermant jusqu'à 12 atomes de carbone, ou un radical aryle ou hétéroaryle à 5 ou 6 chaînons comportant un ou plusieurs hétéroatomes, éventuellement substitués par un ou plusieurs des substituants mentionnés ci-dessus.

Comme hétérocycle préféré, on peut citer entre autres 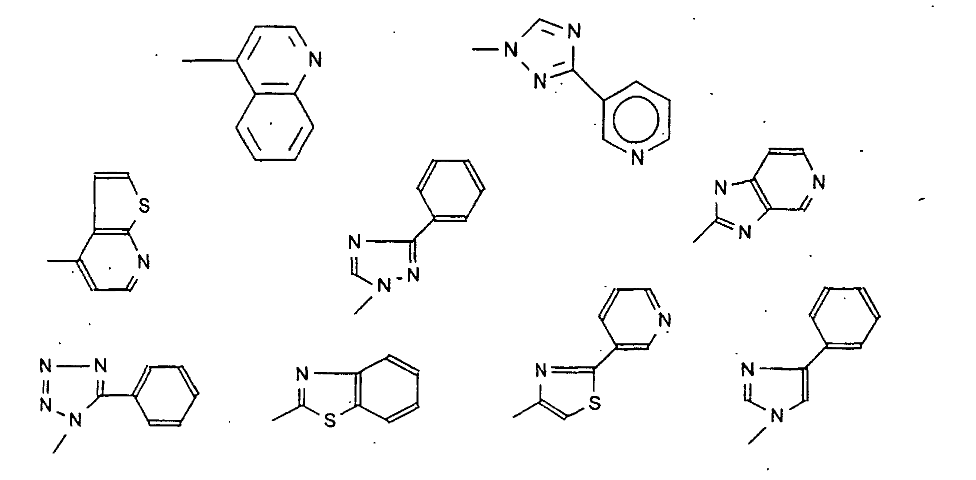 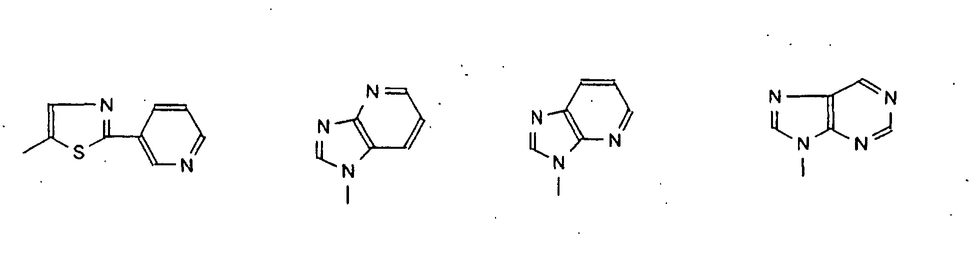 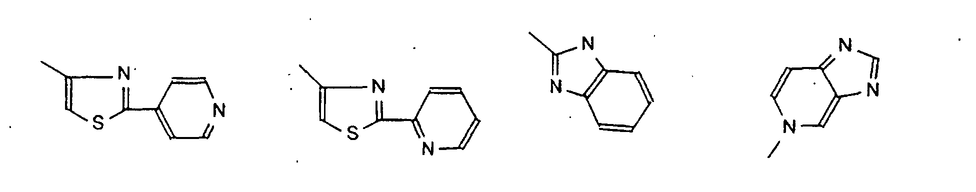 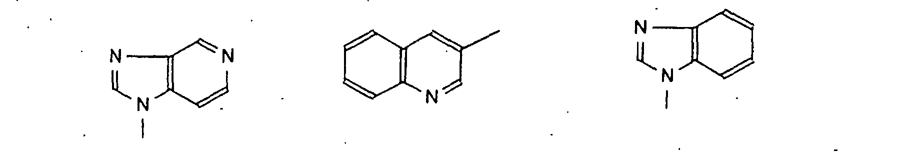 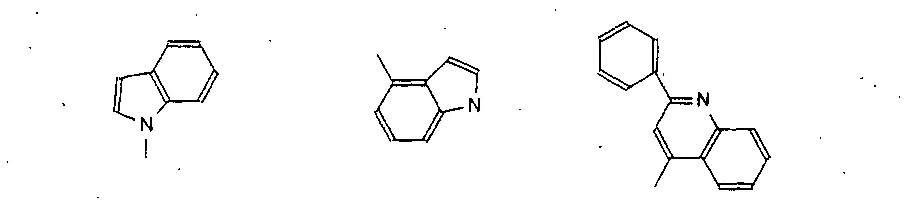 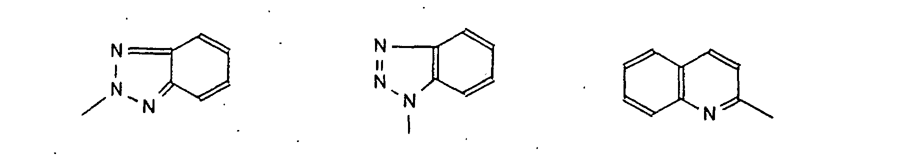 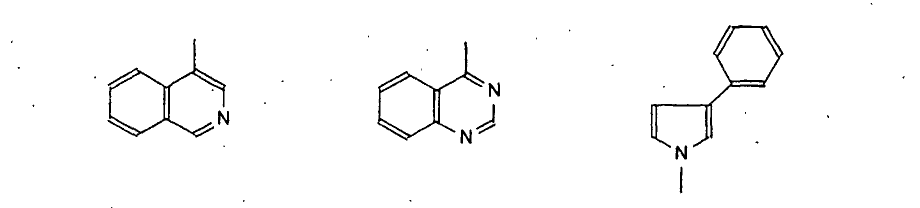 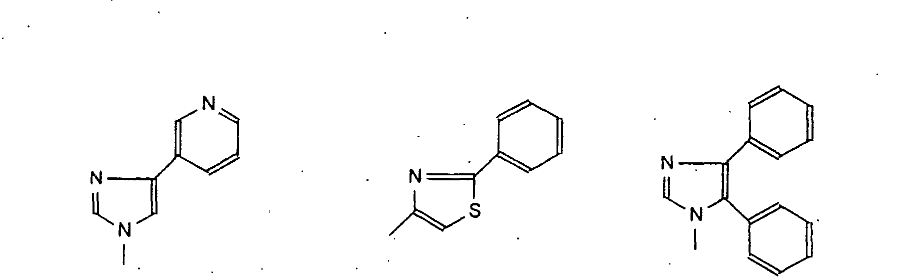 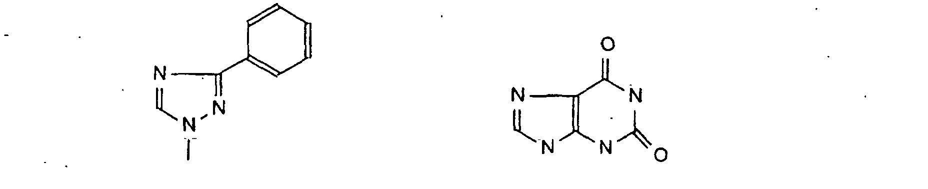 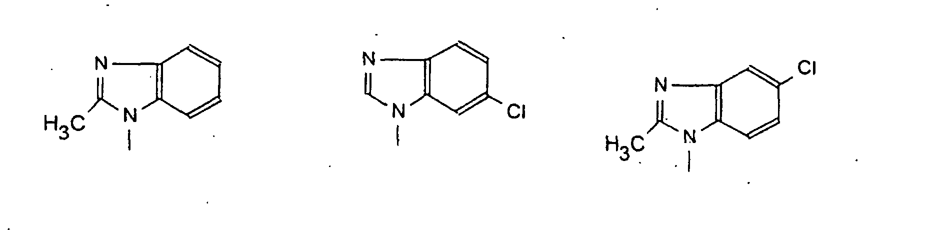 et les radicaux hétérocycliques envisagés dans les demandes de brevets européens 487411, 596802, 676409 et 680967. Ces radicaux hétérocycliques préférés peuvent être substitués par un ou plusieurs groupements fonctionnels.

Hal représente de préférence un atome de fluor, de chlore ou de brome.

Parmi les composés préférés de l'invention, on peut citer ceux dans lesquels Z représente un atome d'hydrogène, ceux dans lesquels W représente un atome d'hydrogène, ceux dans lesquels X représente un radical CH₂, ceux dans lesquels Y représente un radical (CH₂)₃ ou (CH₂)₄, et notamment ceux dans lesquels Ar représence leur radical

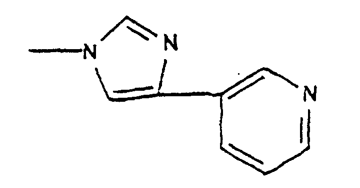

L'invention a tout particulièrement pour objet le produit de l'exemple 1.

Les produits de formule générale (I) possèdent une très bonne activité antibiotique sur les bactéries gram ⊕ telles que les staphylocoques, les streptocoques les pneumocoques.

Les composés de l'invention peuvent donc être utilisés comme médicaments dans le traitement des infections à germe sensibles et notamment, dans celui des staphylococcies, telles que les septicémies à staphilocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumomies, suppurations pulmonaires, les streptococcies telles que les angines aiguës, les otites, les sinusites, la scarlatine, les pneumococcies telles que les pneumonies, les bronchites ; la brucellose, la diphtérie, la gonococcie;

Les produits de la présente invention sont également actifs contre les infections dues à des germes comme Haemophilus influenze, Rickettsies, Mycoplasma pneumoniae, Chlamydia, Legionella, Ureaplasma, Toxoplasma, ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits des exemples et leurs sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 1.

L'invention a également pour objet un procédé de préparation, caractérisé en ce que l'on soumet un composé de formule (II), 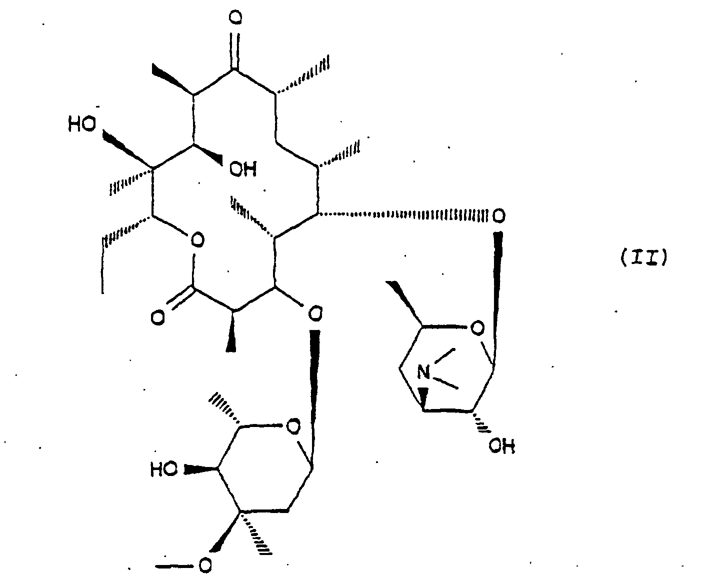 à l'action d'un agent d'hydrolyse du cladinose en milieu aqueux pour obtenir le composé de formule (III), 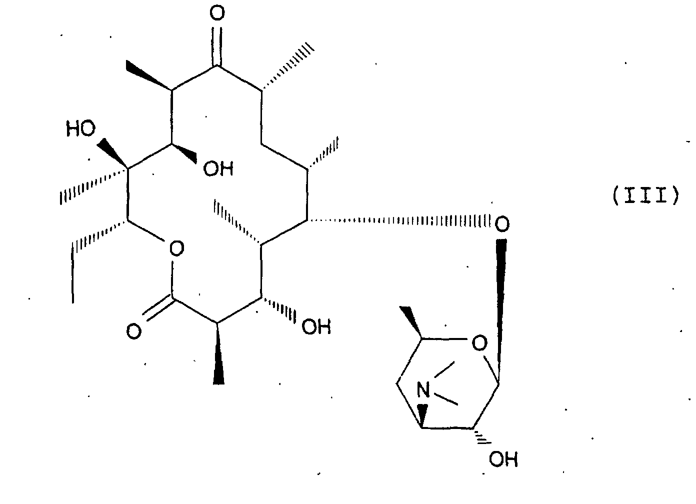 que l'on soumet à l'action d'un agent de blocage de la fonction hydroxyle en 2' pour obtenir un composé de formule (IV) : 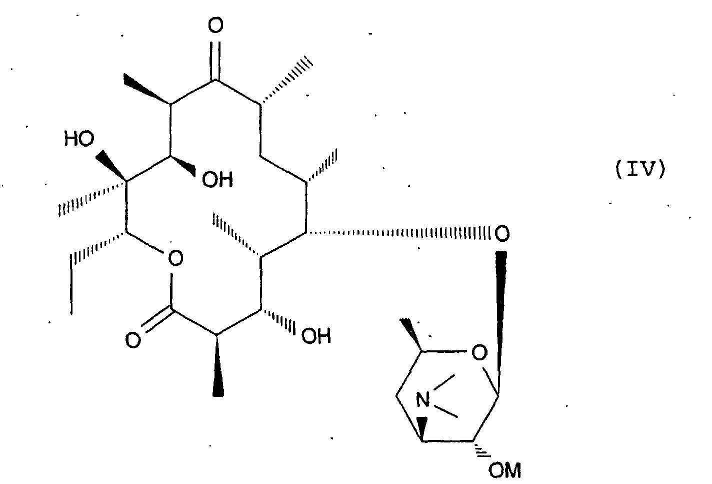 dans laquelle OM représente un groupement hydroxyle bloqué que l'on soumet à l'action d'un agent d'oxydation du groupement hydroxyle en 3 pour obtenir le composé de formule (V) : 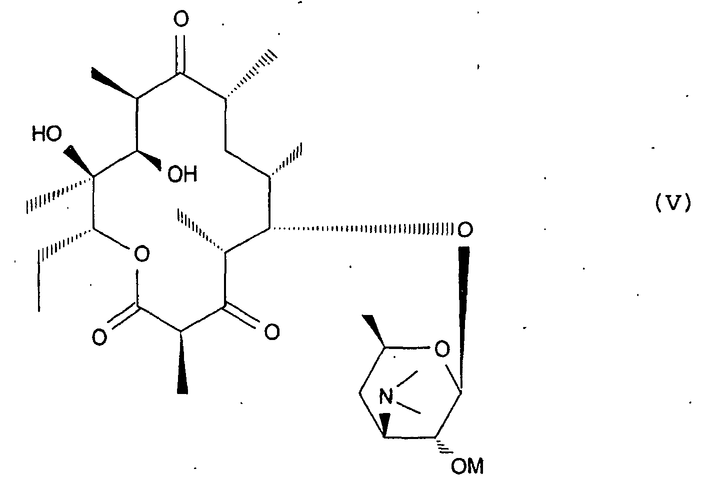 puis si désiré, libère l'hydroxyle en 2' pour obtenir le composé de formule (VI) : 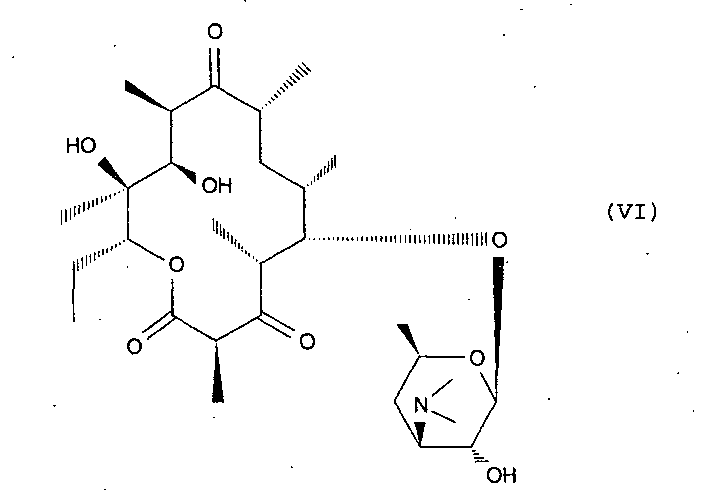 puis si désiré soumet l'un quelconque des produits (V) ou (VI) à l'action d'un agent capable de créer une double liaison en 11, 12 pour obtenir le composé de formule (VII) 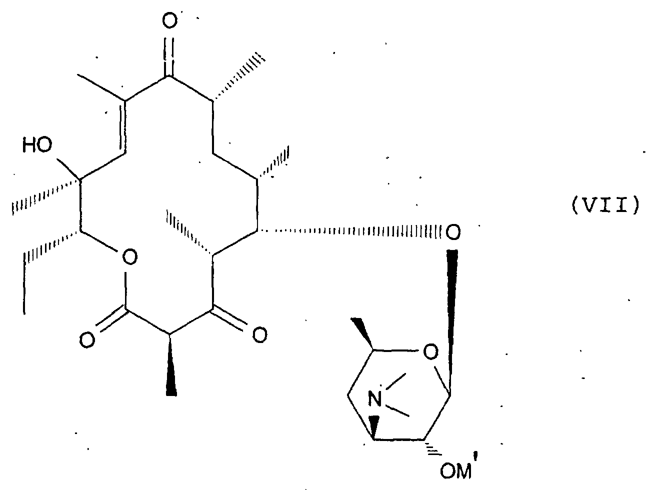 dans laquelle OM' représente un radical hydroxyle libre ou bloqué que l'on soumet à l'action du carbonyldiimidazole pour obtenir le composé de formule (VIII) : 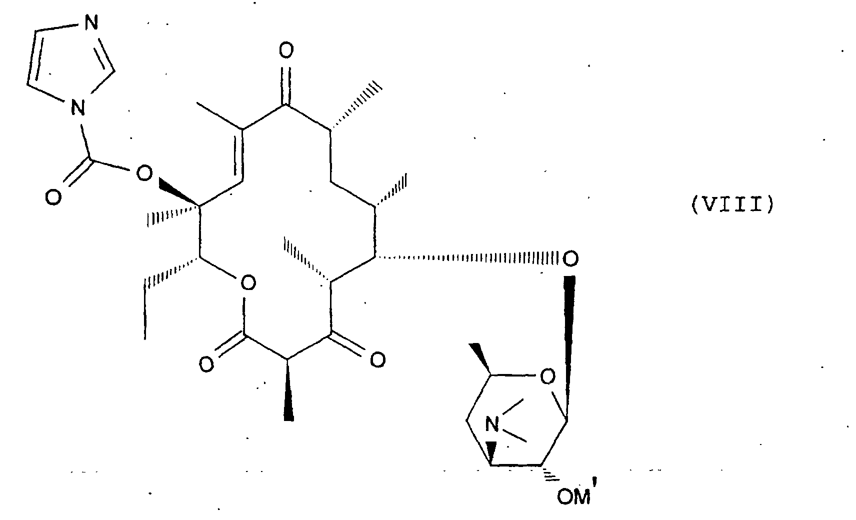 que l'on soumet à l'action d'un composé de formule ArYXNH₂ dans laquelle Y, X et Ar ont la signification indiquée précédemment pour obtenir le composé de formule (IA) correspondant 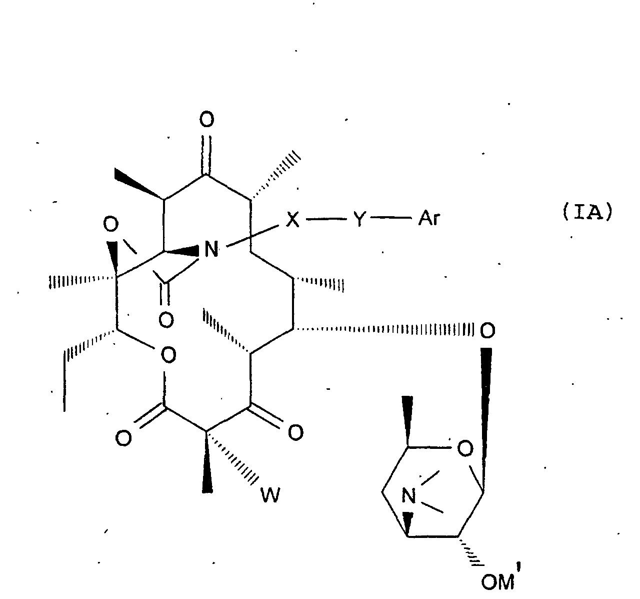 dans laquelle W représenté un atome d'hydrogène que l'on soumet si désiré à l'action d'un agent d'halogénation pour obtenir le composé de formule (IB) dans lequel W représente un atome d'halogène, 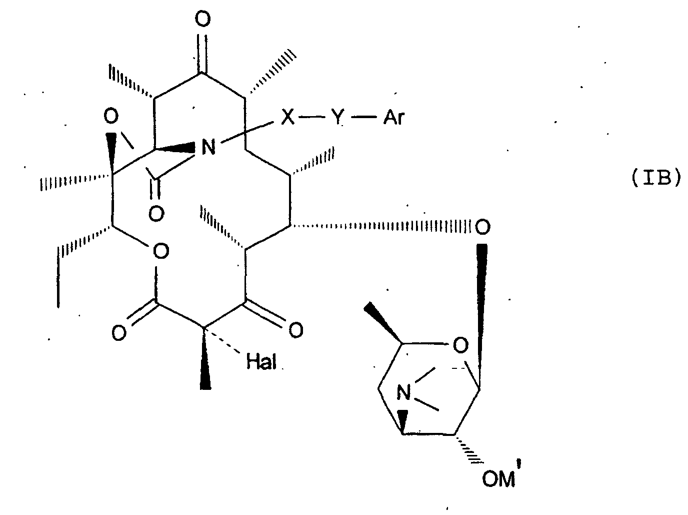 puis si désiré libère l'hydroxyle en 2' et/ou le cas échéant soumet à l'action d'un acide pour en former le sel.

Les composés de formule (II) utilisés comme produits de départ du procédé de l'invention sont des produits connus décrits par exemple dans EP 0216169, EP 41355 et EP 0180415.
- L'hydrolyse du cladinose est réalisée au moyen de l'acide chlorhydrique aqueux ou dans le méthanol.
- Le blocage de l'hydrolyse en 2' est réalisé en utilisant un acide ou un dérivé fonctionnel d'acide par exemple un anhydride d'acide, un halogénure d'acide ou des dérivés du silicium.
- L'oxydation de l'hydroxyle en 3 est effectuée en utilisant des diimides en présence de diméthylsulfoxyde DMSO.
- Le produit de formule (V) ou (VI) est d'abord transformé en carbonate en 11, 12, par action du CDI et du DBU, lequel est transformé en produit (VII) par agitation à 30°C ± 5°C.
- La réaction du composé (VIII) avec ArYXNH₂ a lieu au sein d'un solvant tel que par exemple l'acétonitrile, le diméthyl formamide ou encore le tétrahydrofuranne, le diméthoxyéthane ou le diméthylsulfoxyde.
- L'hydrolyse de la fonction ester en 2' est réalisée à l'aide du méthanol ou de l'acide chlorydrique aqueux.
- La salification est réalisée au moyen d'acide selon les procédés classiques.
- L'halogénation en 2 est par exemple une fluoration, qui peut-être réalisée par utilisation du composé de formule

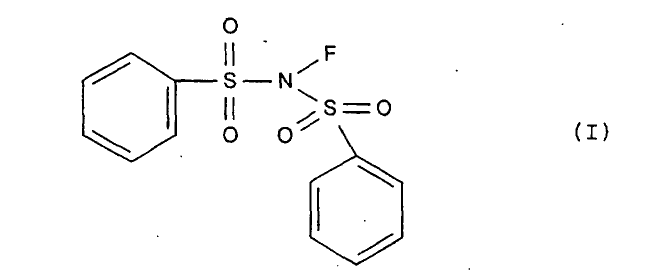

Les composés de formules (III), (IV), (V), (VI), (VII) et (VIII) mis en oeuvre au cours du procédé sont nouveaux et sont en eux-mêmes un objet de la présente invention.

Les exemples suivants illustrent l'invention sans toutefois la limiter.

### EXEMPLE 1 : 3-de-[(2,6-dideoxy-3-C-méthyl-3-O-méthyl-alpha-L-ribo-hexopyranosyl)-oxy]-3-oxo-12,11-[oxycarbonyl[[4-[4- (3-pyridinyl)-1H-imidazol-1-yl]butyl]imino]]-6,11,12-tridéoxyérythromycine et (10S) 3-de-[(2,6-dideoxy-3-C-méthyl-3-O-méthyl-alpha-L-ribo-hexopyranosyl)-oxy]-3-oxo-12,11-[oxycabonyl[[4-[4-(3-pyridinyl)-1H-imidazol-1-yl]butyl]imino]]-6,11,12-tridéoxyérythromycine

### Stade A : 6-déoxy-3-O-de(2,6-didéoxy-3-C-méthyl-.alpha.-L-ribohexo-pyranosyl)-érythromycine

On agite à la température ambiante pendant 3 heures, 100 ml d'eau déminéralisée, 50 ml d'une solution normale d'acide chlorhydrique et 9,58 g d'un mélange de 6,12-didéoxy-érythromycine et de 3"-O-déméthyl-6,12-didéoxy-érythromycine.

On extrait à l'acétate d'éthyle, lave à l'eau, rassemble les phases aqueuses que l'on verse sur une solution d'ammoniaque à 10°C, extrait à l'acétate d'éthyle, lave à l'eau, sèche, filtre et concentre. On purifie le produit obtenu par chromotographie sur silice en éluant avec le mélange CH₂Cl₂/MeOH/NH4OH(95-5-0,5). On recueille les fractions de rf=0,4. On obtient ainsi 0,246 g de produit recherché.

### Stade B : 2'-acétate de 6-déoxy-3-O-de(2,6-didéoxy-3-C-méthyl-3-O-méthyl-alpha-L-ribohexopyranosyl)-érythromycine

On agite pendant 1 H 45 à la température ambiante une solution de 18 ml d'acétate d'éthyle, 0,42 ml d'anhydride acétique et 1,755 g de produit du stade A. On verse dans l'eau, amène à pH 9/10 avec une solution saturée en carbonate de sodium. On extrait à l'acétate d'éthyle, lave avec une solution aqueuse de carbonate de sodium puis à l'eau, sèche, filre et concentre. On obtient 1,826 g de produit recherché.

### Stade C : 2'-acétate de 6-déoxy-3-de[(2,6-didéoxy-3-C-méthyl-3-O-méthyl-alpha-L-ribohexopyranosyl)oxy]-3-oxo-érythromycine

On introduit à la température ambiante 3,45 g de chlohydrate de 1-éthyl-3-(3-diméthylaminopropyl) carbodiimide dans une solution renfermant 55 ml de chlorure de méthylène et 3,5 ml de DMSO. On agite à la température ambiante pendant 25 minutes. On introduit ensuite 1,82 g du produit du stade précédent. On agite pendant 15 minutes. On refroidit à 15°C et ajoute 3,47 g de trifluoroacétate de pyridinium dans 25 ml de chlorure de méthylène. On agite pendant une demi-heure. On purifie par chromatographie sur silice en éluant avec le mélange éther propopylique/isopropanol/TEA (8-1-1). (rf = 0,38). On lave à l'ammoniaque dans l'acétate d'éthyle, sèche et obtient 156 mg de produit recherché.

### Stade D : 6-déoxy-3-O-de-[(2,6-didéoxy-3-C-méthyl-3-O-méthyl-alpha-L-ribohexopyranosyl)-oxy]-3-oxo-érythromycine

On agite à 5°C pendant 40 heures une solution renfermant 0,5 ml de méthanol et 61 mg de produit du stade C. On amène à la température ambiante, agite pendant 8 heures à température ambiante et évapore le solvant. On chromatographie le produit obtenu sur silice, éluant CH₂Cl₂/isopropanol/NH₄OH(94-4-0,5). On obtient 14 mg de produit recherché.

### Stade E : 2'-acétate et 11,12-carbonate cyclique de 6-déoxy-3-de[(2,6-didéoxy-3-C-méthyl-3-O-méthyl-.alpha.-L-ribo-hexopyra-nosyl)oxy]-3-oxo-érythromycine et 2'-acétate de 10,11-didéhydro-6,11-dideoxy-3-de[2,6-didéoxy-3-C-méthyl-3-O-méthyl-alpha-L-ribo-hexopyranosyl)oxy]-3-oxo-érythromycine

On agite pendant 5 heures à la température ambiante une solution de 3,5 ml d'acétate d'éthyle, 0,346 g de produit du stade C, 9µl de DBU, 0,127 g de 1,1' carbonyldiimidazole. On l'agite ensuite 15 heures à 30°C. On verse sur l'eau, extrait à l'acétate d'éthyle, lave à l'eau, sèche, filtre et concentre. On obtient 0,301 g de produit que l'on chromatographie sur silice en éluant avec le mélange chlorure de méthylène, isopropanol, ammoniaque 95-5-05. On concentre les fractions homogènes en CCM, reprend à l'acétate d'éthyle, sèche, filtre et concentre. On obtient 0,162 g de produit recherché.

### Stade F : 2'-acétate et 12-[(1H-imidazol-1-yl)carboxylate]de 10,11-didéhydro-6,11-didéoxy-3-de[(2,6-didéoxy-3-C-méthyl-3-O-méthyl-alpha-L-ribo-hexopyranosyl)-oxy]-3-oxo-érythromycine

On agite à 0°C pendant 3 heures une solution renfermant 2 ml de THF, 0,155 g de produit du stade précédent, 6 µl de DBU et 0,064 g de 1,1' carbonyldiimidazole. On agite à 10°C pendant 15 heures. On verse sur l'eau, extrait à l'acétate d'éthyle; lave à l'eau, sèche, filtre et concentre. On obtient ainsi le produit recherché.

### Stade G : 3-de[2,6-didéoxy-3-C-méthyl-3-O-méthyl-alpha-L-ribo-hexopyranosyl)-oxy]-3-oxo-12,11-[oxycarbonyl[[4-[4-(3-pyridinyl)-1H-imidazol-1-yl]-butyl]-imino]]-6,11,12-tridéoxy-érythromycine (produit A) et (10S) 3-de[2,6-didéoxy-3-C-méthyl-3-O-méthyl-.alpha.-L-ribo-hexopyranosyl)oxy]-3-oxo-12,11-[oxycarbonyl[[4-[4-(3-pyridinyl)-1H-imidazol-1-yl]butyl]imino]]-6,11,12-tridéoxy-érythromycine (produit B)

On agite pendant 4 heures à 60°C une solution renfermant 1,5 ml d'acétonitrile, 0,15 ml d'eau, 0,17 g de produit du stade précédent et 0,199 g de 4-(3-pyridinyl)1H-imidazol-1-butanamine. On verse sur l'eau, extrait à l'acétate d'éthyle, lave à l'eau, sèche, filtre et concentre. On obtient 0,184 g de produit que l'on verse dans une solution de 2 ml de méthanol et 25 µl de DBU. On agite pendant 16 heures à la température ambiante. On évapore le méthanol, chromatographie sur silice en éluant avec le mélange chlorure de méthylène/méthanol/ammoniaque(93-7-0,5). On recueille la fraction de rf = 0,38, lave à l'ammoniaque dans l'acétate d'éthyle, sèche sur sulfate de magnésium. On obtient 37 mg de produit A. On recueille aussi la fraction de rf = 0,36, la purifie à nouveau par chromatographie sur silice en éluant avec le mélange chlorure de méthylène/méthanol/ammoniaque/(93-7-1). On lave avec le mélange chlorure de méthylène, isopropanol, ammoniaque 9-1-0,5-. On concentre, reprend le résidu au chlorure de méthylène, sèche, filtre et concentre. On obtient 13 mg de produit B.

### EXEMPLES DE COMPOSITIONS PHARMACEUTIQUES

On a préparé des comprimés renfermant :

| | |
|---|---|
| **Produit de l'exemple 1** | 150 mg |
| Excipient q.s.p. | 1 g |
| Détail de l'excipient : amidon, talc, stéarate de magnésium | |

On a également préparé des solutions infectables à partir des produits salifiés.

### ETUDE PHARMACOLOGIQUE DES PRODUITS DE L'INVENTION

### A - Méthode des dilutions en milieu liquide

On a préparé une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de 24 heures à l'étuve à 37°C, l'inhibition de la croissance est appréciée par transillumination de ce qui permet de déterminer les concentrations minimales inhibitrices (C.M.I.) exprimées en 5 microgrammes/cm3.

Les résultats suivants ont été obtenus avec le produit de l'exemple : (lecture après 24 heures)

| | | |
|---|---|---|
| S. aureus | 011UC4 | 0.300 |
| S. agalactiae | 02B1HT1 | <+0.02 |
| E. faecalis | 02D2UC1 | 0.080 |
| E. faecium | 02D3HT1 | 0.040 |
| Streptococcus gr;G | 02GOGR5 | 0.040 |
| S. mitis | 02MitCB1 | 0.040 |
| S. pyogenes | 02A1SJc | |
| S. agalactiae | 02B1SJ1c | 2.500 |
| Streptococcus gr.G | 02Gogr4c | |
| S.mitis | 02MitGR16i | 0.300 |
| S. pneumoniae | 032UC1 | <=0.02 |
| S. pneumoniae | 030GR20 | 0.600 |
| S. pneumoniae | 030SJ5i | 0.600 |
| S. pneumoniae | 030CR18c | |
| S. pneumoniae | 030PW23c | 0.300 |
| S. pneumoniae | 030RO1i | 0.600 |

## Revendications

1. Les composés de formule (I) 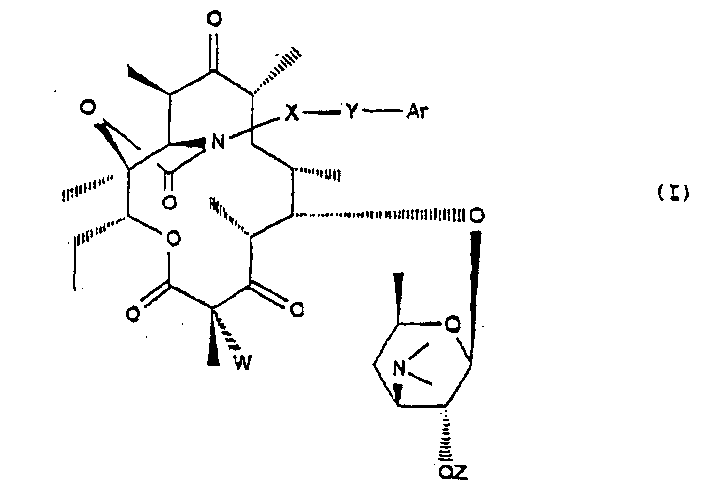 dans lesquels
- X représente un radical (NH) a, CH₂ ou SO₂ ou un atome d'oxygène, a représente le nombre 0 ou 1,
- Y représente un radical (CH₂)m-(CH=CH)n-(CH₂)o avec m+n+o ≤ 8, n = O ou 1,
- Ar représentant un radical aryle ou hétéroaryle, éventuellement substitué
- W représente un atome d'hydrogène ou un atone d'halogène
- Z représentant un atome d'hydrogène ou le reste d'un acide ainsi que leurs sels d'addition avec les acides.

2. Les composés de formule (I) définis à la revendication 1 dans lesquels Z représente un atome d'hydrogène

3. Les composés de formule (I) définis à l'une quelonque des revendications 1 et 2 dans lesquels W représente un atome d'hydrogène

4. Les composés de formule (I) définis à l'une quelconque des revendications 1, 2 et 3 dans lesquels X représente un radical CH₂

5. Les composés de formule (I) définis à l'une quelconque des revendications 1 à 4 dans lesquels Y représente un radical (CH₂)₃ ou (CH₂)₄

6. Les composés de formule (I) définis à l'une quelconque des revendications 1 à 5, **caractérisés en ce que** Ar représente le radical

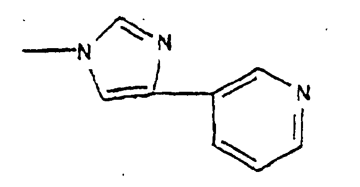

7. A titre de produit chimique défini à la revendication 1 : 1s 3-de[(2,6-dideoxy-3-C-méthyl-3-O-méthyl-alpha-L-ribo-hexopyranosyl)oxy]-3-oxo-12,11-[oxycarbonyl[[4-(4-(3-pyridinyl)-1H-imidazol-1-yl]butyl]imino]]-6,11,12-tridéoxy-érythromycine

8. A titre de médicaments, les composés selon l'une quelconque des revendications 1 à 6, ainsi que leurs sels pharmaceutiquement acceptables

9. A titre de médicaments, le composé selon la revendication 7, ainsi que ses sels pharmaceutiquement acceptables

10. Les compositions pharmaceutiques refermant comme médicaments au moins un médicament selon la revendication 8 ou 9.

11. Procédé de préparation des composés de formule (I) définis à l'une quelconque des revendications 1 à 7, **caractérisés en ce que** l'on soumet un composé de formule (II), 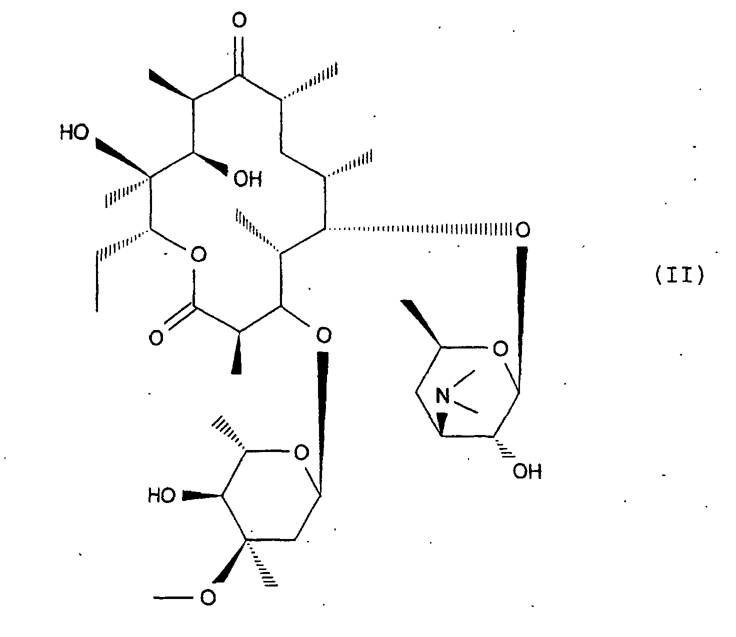 à l'action d'un agent d'hydrolyse du cladinose en milieu aqueux pour obtenir le composé de formule (III) : 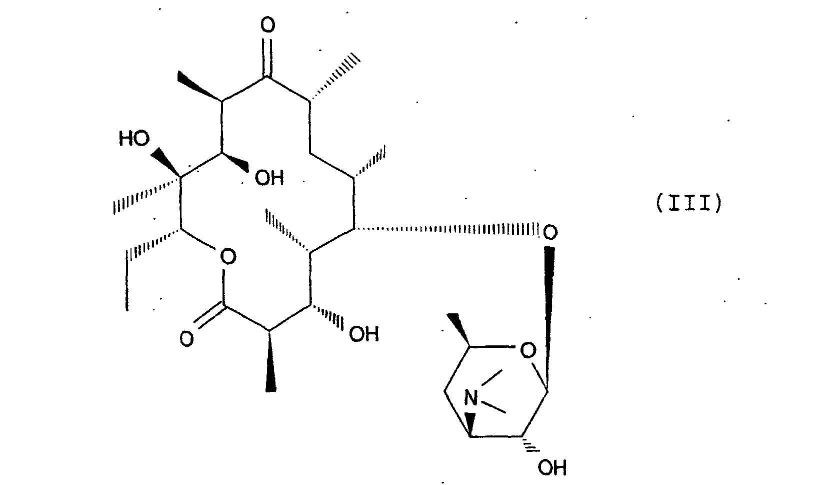 que l'on soumet à l'action d'un agent de blocage de la fonction hydroxyle en 2' pour obtenir un composé de formule (IV) : 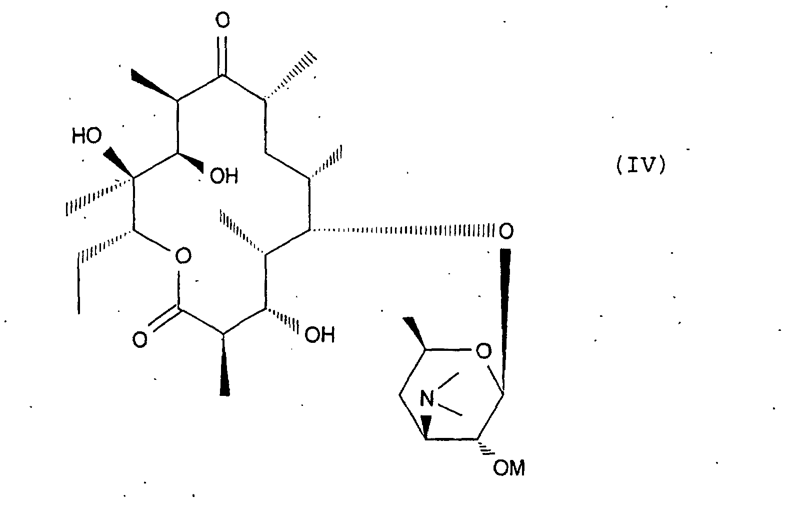 dans laquelle OM représente un groupement hydroxyle bloqué que l'on soumet à l'action d'un agent d'oxydation du groupement hydroxyle en 3 pour obtenir le composé de formule (V) : 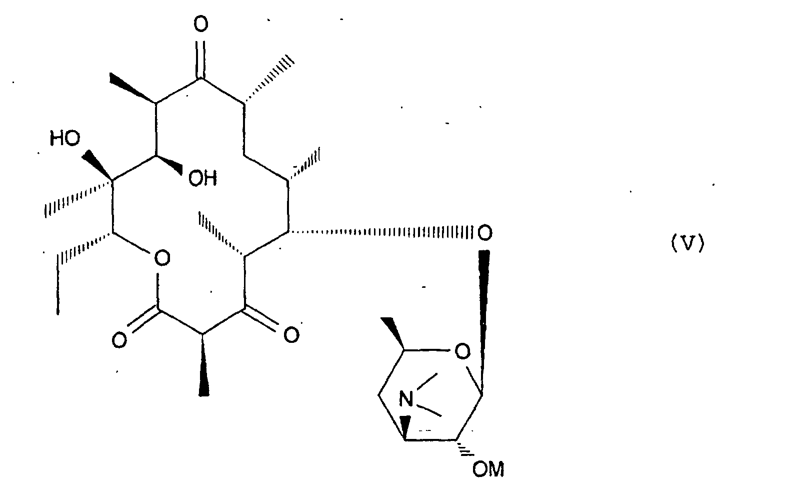 puis si désiré libère l'hydroxyle en 2' pour obtenir le composé de formule (VI) 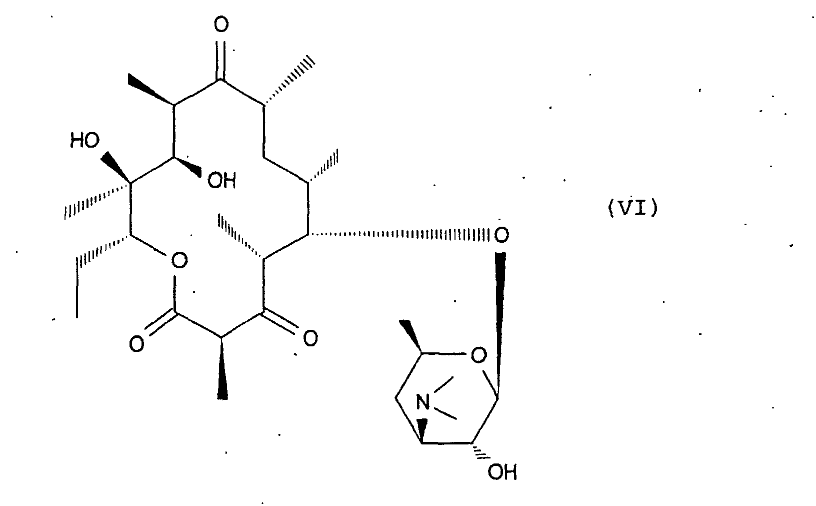 puis si désiré soumet l'un quelconque des produits (V) ou (VI) à l'action d'un agent capable de créer une double liaison en 11, 12 pour obtenir le composé de formule (VII) : 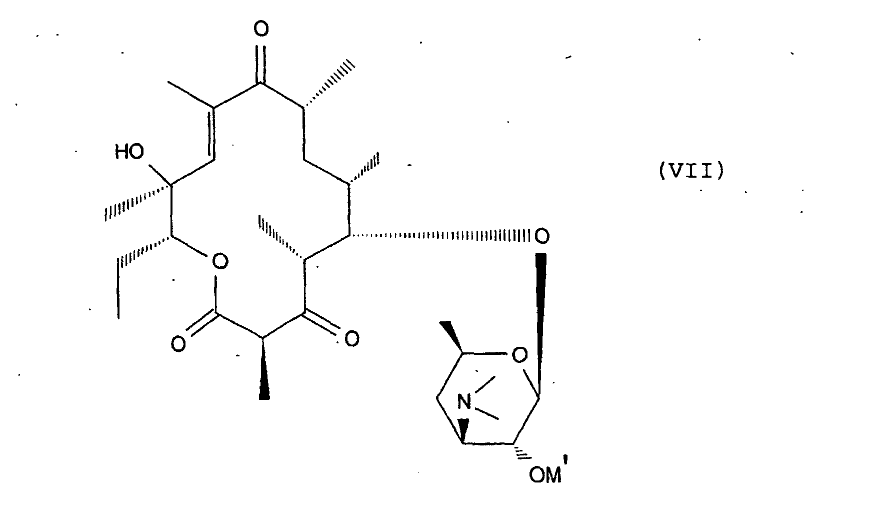 dans laquelle OM' représente un radical hydroxyle libre ou bloqué que l'on soumet à l'action du carbonyldiimidazole pour obtenir le composé de formule (VIII) : 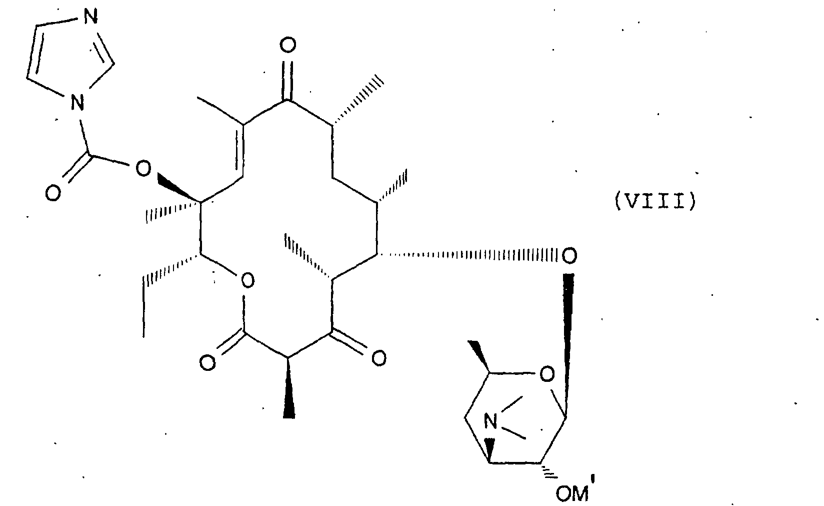 que l'on soumet à l'action d'un composé de formule ArYXNH₂ dans laquelle Y, X, et Ar ont la signification indiquée à la revendication 1, pour obtenir le composé de formule (IA) correspondant 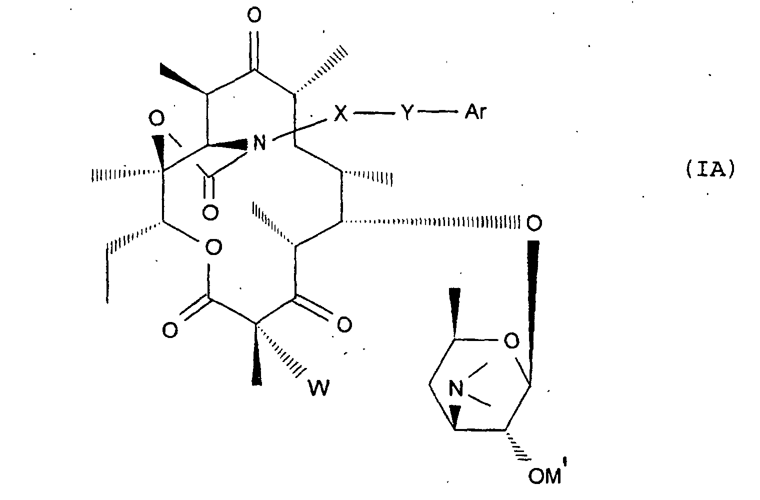 dans laquelle W représente un atome d'hydrogène que l'on soumet si désiré à l'action d'un agent d'halogénation pour obtenir le composé de formule (IB) dans lequel W représente un atome d'halogène, 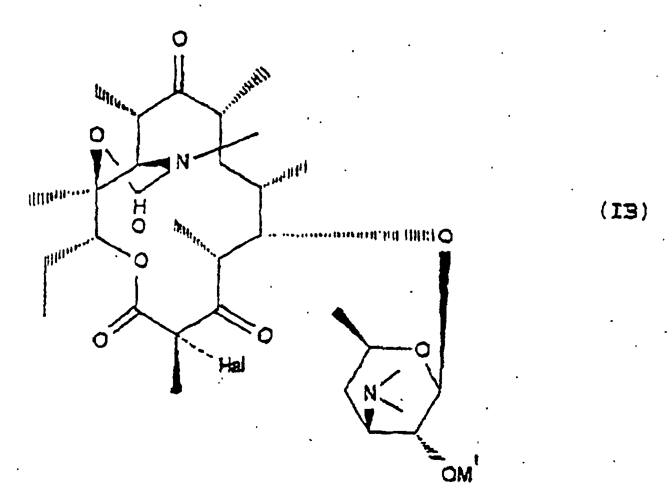 puis si désiré libère l'hydroxyle en 2' et/ou le cas échéant soumet à l'action d'un acide pour en former le sel

12. A titre de produits chimiques, les composés de formule (V), (VI), (VII) et (VIII) définis à la revendication 11.

## Claims

1. The compounds of formula (I) 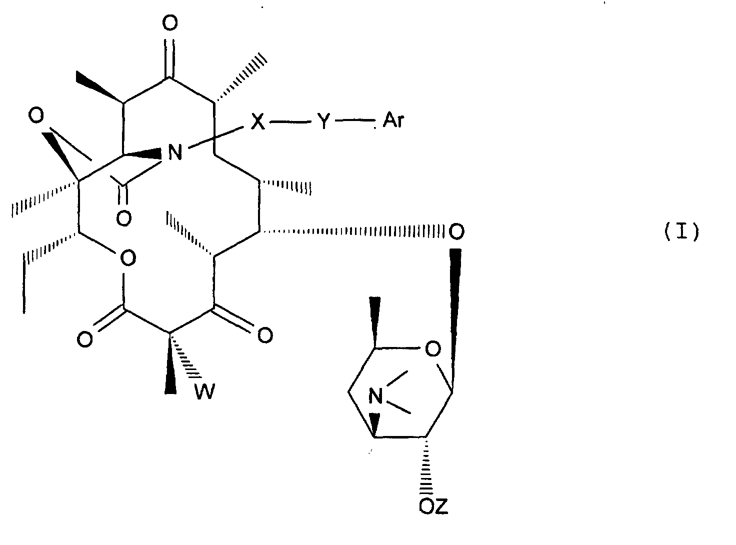 in which
- X represents an (NH)a, CH₂ or SO₂ radical or an oxygen atom, a represents the number 0 or 1,
- Y represents a (CH₂)m-(CR=CR)n-(CH₂)o radical with m+n+o ≤8, n = 0 or 1,
- Ar represents an aryl or heteroaryl radical, optionally substituted
- W represents a hydrogen atom or a halogen atom
- Z represents a hydrogen atom or the remainder of an acid as well as their addition salts with acids.

2. The compounds of formula (I) defined in claim 1, in which Z represents a hydrogen atom.

3. The compounds of formula (I) defined in any one of claims 1 and 2, in which W represents a hydrogen atom.

4. The compounds of formula (I) defined in any one of claims 1, 2 and 3, in which X represents a CH₂ radical.

5. The compounds of formula (I) defined in any one of claims 1 to 4, in which Y represents a (CH₂)₃ or (CH₂)₄ radical.

6. The compounds of formula (I) defined in any one of claims 1 to 5, **characterized in that** Ar represents the 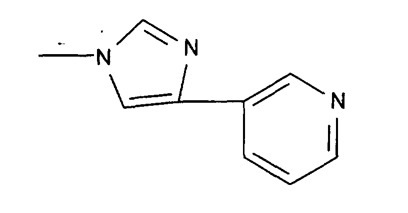 radical.

7. As a chemical product defined in claim 1: 3-de[(2,6-dideoxy-3-C-methyl-3-O-methyl-alpha-L-ribo-hexopyranosyl)oxy]-3-oxo-12,11-[oxycarbonyl[[4-(4-(3-pyridinyl)-1H-imidazol-1-yl]butyl]imino]]-6,11,12-trideoxy-erythromycin.

8. As medicaments, the compounds according to any one of claims 1 to 6, as well as their pharmaceutically acceptable salts

9. As a medicaments, the compound according to claim 7, as well as its pharmaceutically acceptable salts

10. The pharmaceutical compositions containing at least one medicament according to claim 8 or 9 as medicaments.

11. Process for the preparation of compounds of formula (I) defined in any one of claims 1 to 7, **characterized in that** a compound of formula (II): 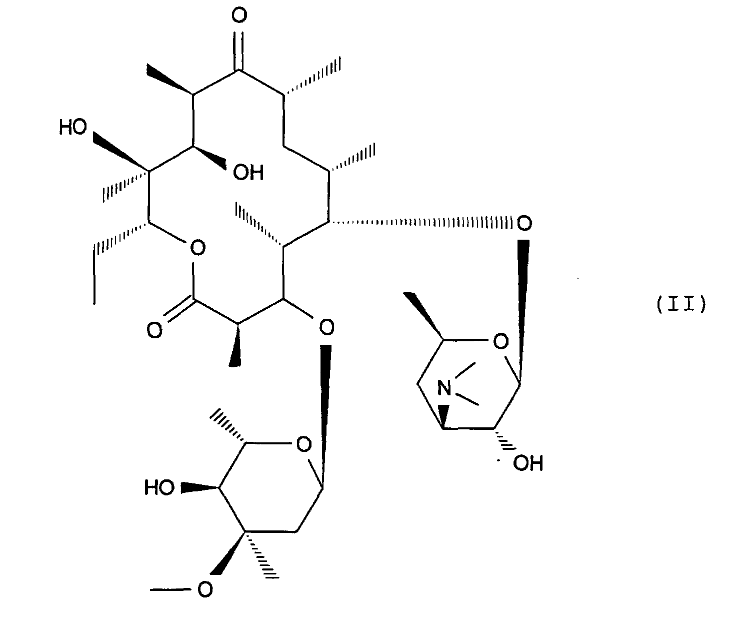 is subjected to the action of a cladinose hydrolysis agent in aqueous medium in order to obtain the compound of formula (III): 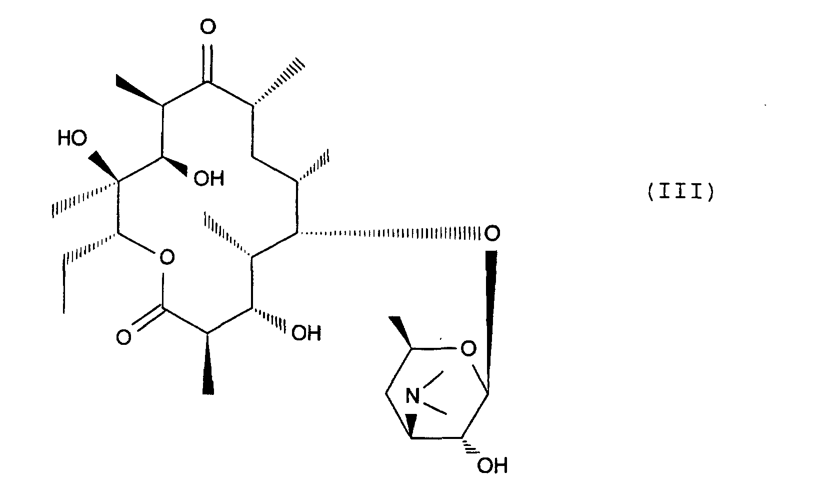 which is subjected to the action of a blocking agent of the hydroxyl function in position 2' in order to obtain a compound of formula (IV): 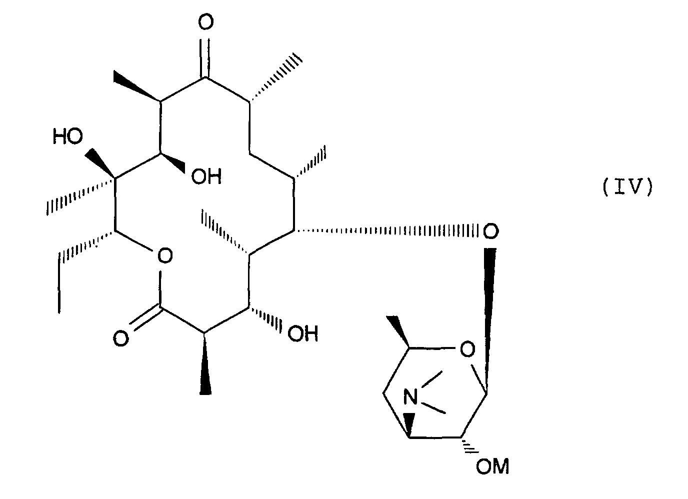 in which OM represents a blocked hydroxyl group which is subjected to the action of an oxidizing agent of the hydroxyl group in position 3 in order to obtain the compound of formula (V): 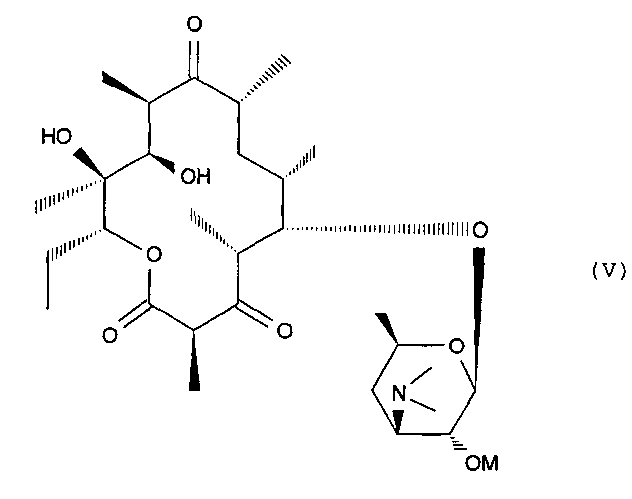 then if desired the hydroxyl in position 2' is released in order to obtain the compound of formula (VI): 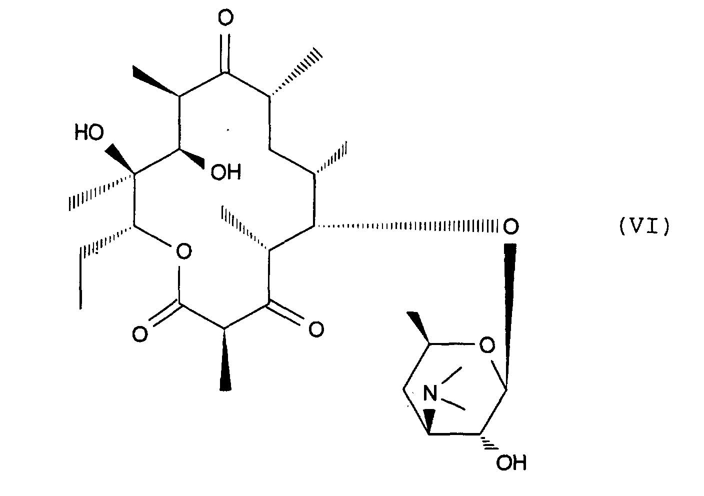 then if desired any one of products (V) or (VI) is subjected to the action of an agent capable of creating a double bond in position 11, 12 in order to obtain the compound of formula (VII): 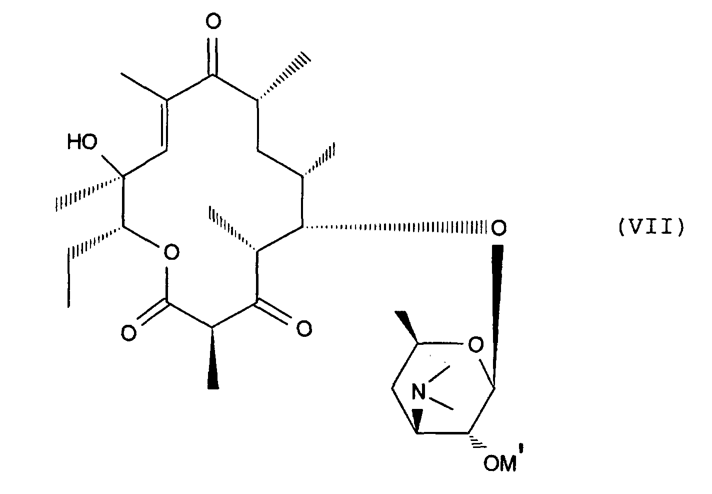 in which OM' represents a free or blocked hydroxyl radical which is subjected to the action of carbonyldiimidazole in order to obtain the compound of formula (VIII): 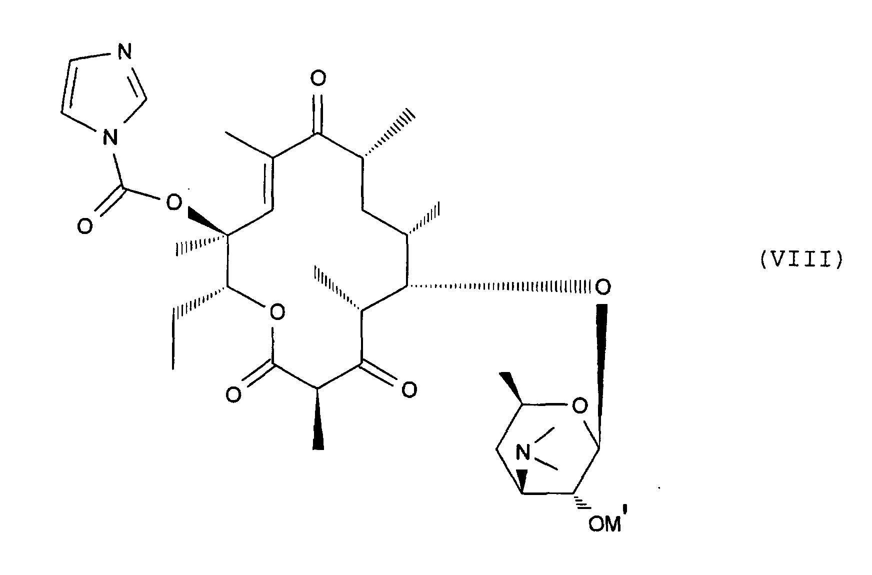 which is subjected to the action of a compound of formula ArYXNH₂ in which Y, X, and Ar have the meaning indicated in claim 1, in order to obtain the corresponding compound of formula (IA) 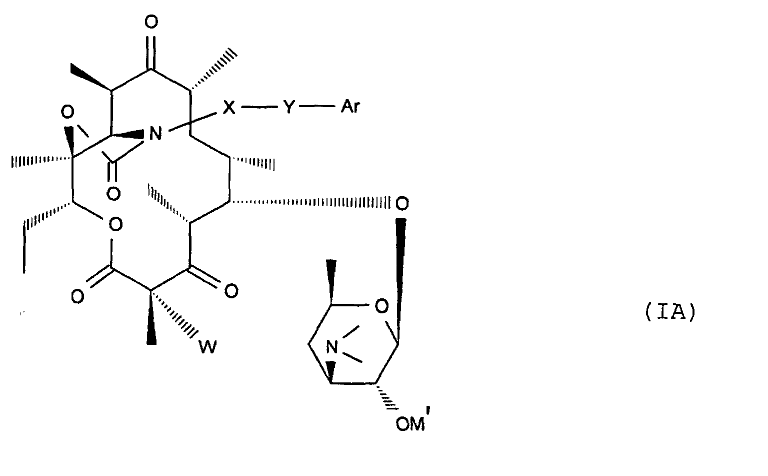 in which W represents a hydrogen atom which is subjected if desired to the action of a halogenation agent in order to obtain the compound of formula (IB) in which W represents a halogen atom, 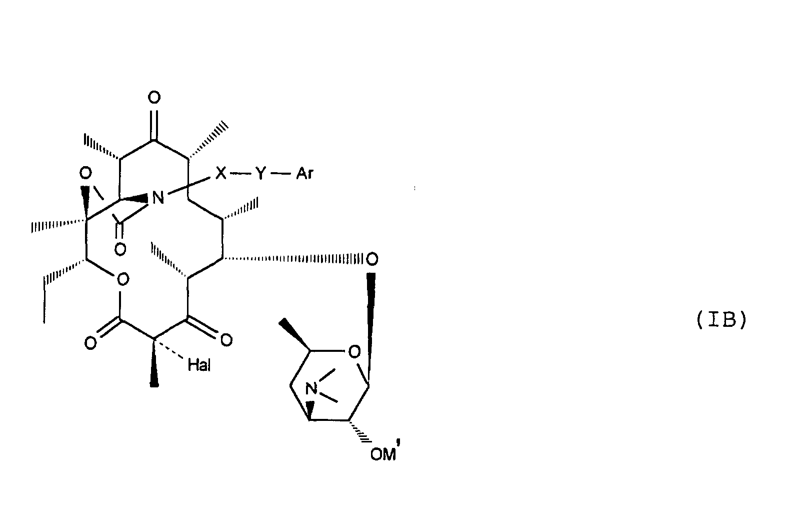 then if desired the hydroxyl in position 2' is released and/or if appropriate subjected to the action of an acid in order to form the salt.

12. As chemical products, the compounds of formula (III), (IV), (V), (VI), (VII) and (VIII) defined in claim 11.

## Patentansprüche

1. Verbindungen der Formel (I) 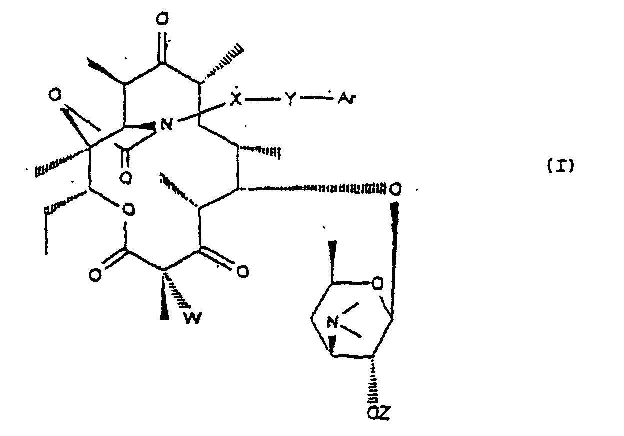 in der
- X einen Rest (NH)a, CH₂ oder SO₂ oder ein Sauerstoffatom darstellt und a die Zahl 0 oder 1 ist,
- Y einen Rest (CH₂)m-(CH=CH)n-(CH₂)o bedeutet mit m+n+o ≤ 8 und n = 0 oder 1,
- Ar einen Rest Aryl oder Heteroaryl darstellt, gegebenenfalls substituiert,
- W ein Wasserstoffatom oder ein Halogenatom ist,
- Z ein Wasserstoffatom oder den Rest einer Säure bedeutet, sowie ihre Additionssalze mit Säuren.

2. Verbindungen der Formel (I) wie in Anspruch 1 definiert, worin Z ein Wasserstoffatom darstellt.

3. Verbindungen der Formel (I) wie in irgendeinem der Ansprüche 1 und 2 definiert, worin W ein Wasserstoffatom darstellt.

4. Verbindungen der Formel (I) wie in irgendeinem der Ansprüche 1, 2 und 3 definiert, worin X einen Rest CH₂ darstellt.

5. Verbindungen der Formel (I) wie in irgendeinem der Ansprüche 1 bis 4 definiert, worin Y einen Rest (CH₂)₃ oder (CH₂)₄ darstellt.

6. Verbindungen der Formel (I) wie in irgendeinem der Ansprüche 1 bis 5 definiert, **dadurch gekennzeichnet, daß** Ar den Rest 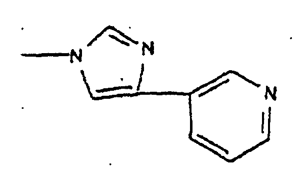 darstellt.

7. Als chemisches Produkt wie in Anspruch 1 definiert 3-De-[(2,6-Dideoxy-3-C-methyl-3-O-methyl-alpha-L-ribohexopyranosyl)-oxy]-3-oxo-12,11-{oxycarbonyl-[[4-(4-(3-pyridinyl)-1H-imidazol-1-yl)-butyl]-imino]}-6,11,12-trideoxy-erythromycin

8. Als Arzneimittel die Verbindungen nach irgendeinem der Ansprüche 1 bis 6 sowie ihre pharmazeutisch akzeptablen Salze.

9. Als Arzneimittel die Verbindung nach Anspruch 7 sowie ihre pharmazeutisch akzeptablen Salze.

10. Pharmazeutische Zusammensetzungen, umfassend als Arzneimittel mindestens ein Arzneimittel nach Anspruch 8 oder 9.

11. Verfahren zur Herstellung der Verbindungen der Formel (I) wie in irgendeinem der Ansprüche 1 bis 7 definiert, **dadurch gekennzeichnet, daß** man eine Verbindung der Formel (II) 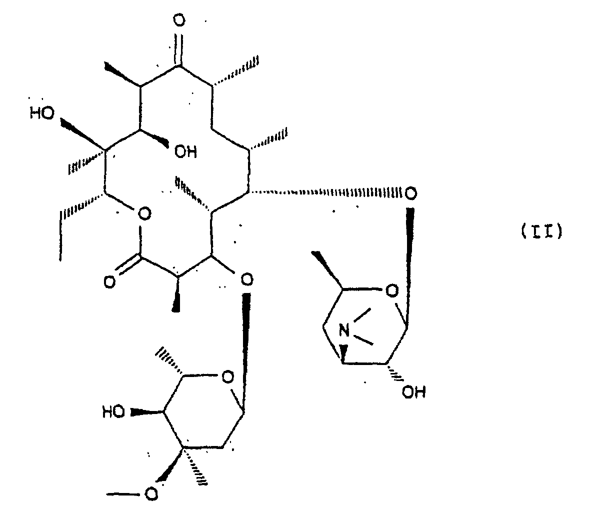 der Einwirkung eines Hydrolysemittels der Cladinose im wäßrigen Medium unterzieht, um die Verbindung der Formel (III) 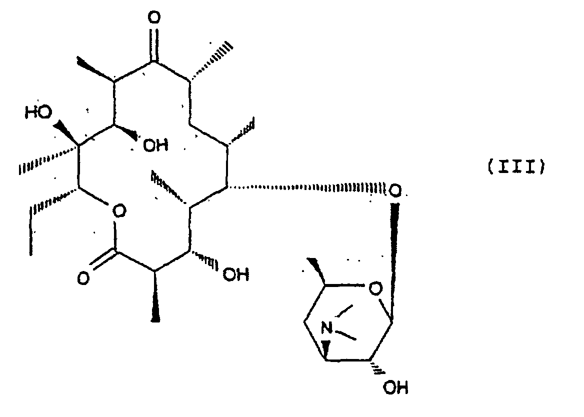 zu erhalten, die man der Einwirkung eines Blockierungsmittels für die Hydroxylfunktion in 2' unterzieht, um eine Verbindung der Formel (IV) 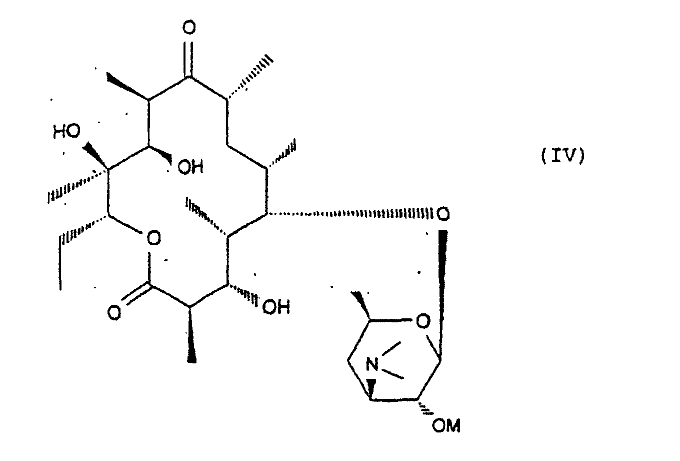 zu erhalten, in der OM eine blockierte Hydroxylgruppe'darstellt, die man der Einwirkung eines Oxidationsmittels für die Hydroxylgruppe in 3 unterzieht, um die Verbindung der Formel (V) 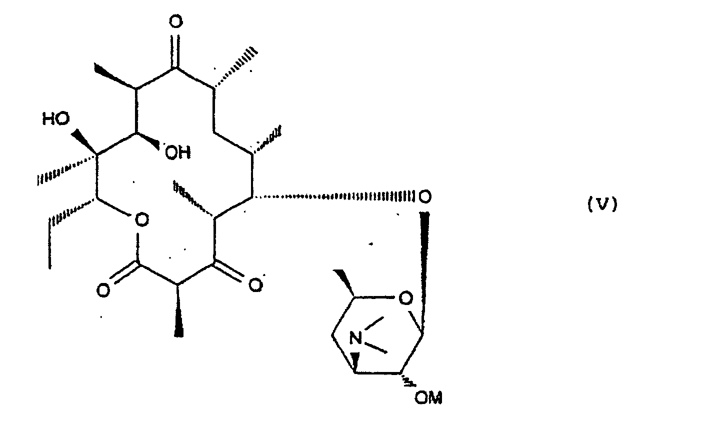 zu erhalten, bei der man anschließend, wenn erwünscht, das Hydroxyl in 2' freisetzt, um die Verbindung der Formel (VI) 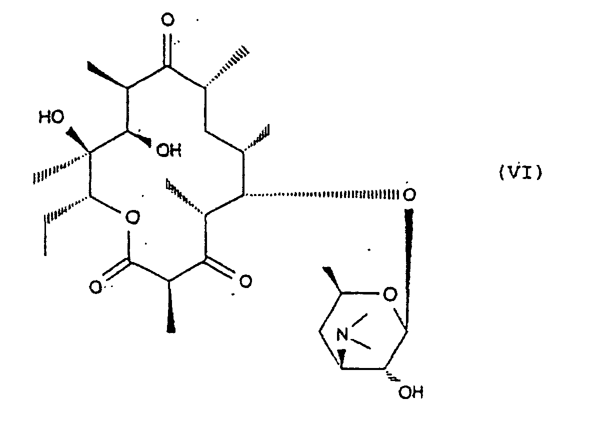 zu erhalten, und man anschließend, wenn erwünscht, irgendeines der Produkte (V) oder (VI) der Einwirkung eines Mittels unterzieht, das geeignet ist, eine Doppelbindung in 11,12 zu erzeugen, um die Verbindung der Formel (VII) 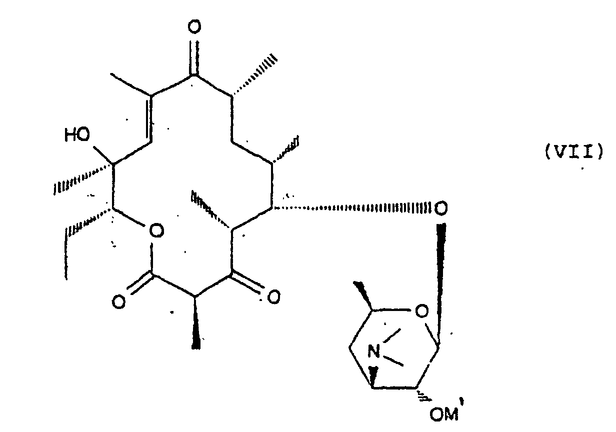 zu erhalten, in der OM' einen freien oder blockierten Rest Hydroxyl darstellt, die man der Einwirkung von Carbonyldiimidazol unterzieht, um die Verbindung der Formel (VIII) 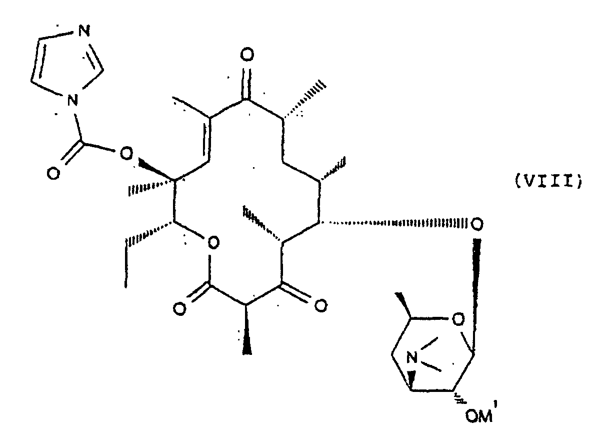 zu erhalten, die man der Einwirkung einer Verbindung der Formel ArYXNH₂, in der Y, X und Ar die in Anspruch 1 angegebene Bedeutung besitzen, unterzieht, um die Verbindung der Formel (IA) 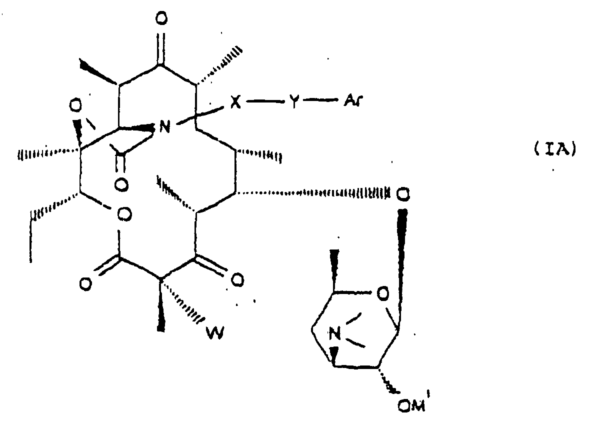 zu erhalten, in der W ein Wasserstoffatom darstellt, die man, wenn erwünscht, der Einwirkung eines Mittels zur Halogenierung unterzieht, um die Verbindung der Formel (IB), worin W ein Halogenatom darstellt, 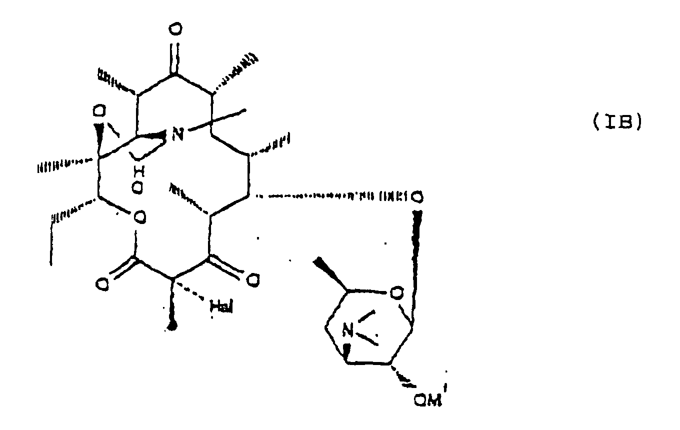 zu erhalten, bei der man anschließend, wenn erwünscht, das Hydroxyl in 2' freisetzt und/oder gegebenenfalls der Einwirkung einer Säure unterzieht, um daraus das Salz zu bilden.

12. Als chemische Produkte die Verbindungen der Formeln (V), (VI), (VII) und (VIII) wie in Anspruch 11 definiert.
